# EUROPEAN PATENT SPECIFICATION

(11) **EP 2 313 322 B1**
(45) Date of publication and mention of the grant of the patent: **20.04.2016**
(21) Application number: 09797257.4
(22) Date of filing: 08.07.2009
(51) Int. Cl.: B65D 25/28, A61J 1/00, B65D 1/00, B65D 1/40, A61B 10/00

(54) **A SPECIMEN CONTAINER**
PROBENBEHÄLTER
CONTENANT À ÉCHANTILLON

(30) Priority: 15.07.2008 AU 2008903597; 12.11.2008 AU 2008905824
(43) Date of publication of application: 27.04.2011
(73) Proprietor: Jawkaumi Pty Ltd, Harrismith, Western Australia 6361 (AU)
(72) Inventor: KHOURY, Edward, Joseph, Perth, Western Australia 6150 (AU)
(74) Representative: Adamson Jones
(86) International application number: PCT/AU2009/000885
(87) International publication number: WO 2010/006359

(56) References cited:
- WO-A2-2005/112743
- US-A- 3 318 493
- US-A- 6 126 035
- US-A1- 2005 011 854
- US-B1- 6 485 438
- US-B1- 6 485 438
- US-B1- 6 761 282
- US-B1- 6 799 694

## Description

### Field of the Invention

The present invention relates to a specimen container.

### Background to the Invention

Containers used to collect samples of urine for medical analysis commonly comprise a small cylindrical container having a lid. While such small containers are well suited to storage and transport of specimens and are relatively inexpensive to produce in the large quantities required, they can present difficulties in the process of providing the sample.

The usual process comprises the patient simply holding the container around the outer surface while providing the sample. Holding the container in this manner to effectively collect the sample while keeping the hands out of the way can be difficult for women, and in particular pregnant women who are often required to provide urine samples for testing.

US 6485438 discloses a disposable urine specimen collection cup with a two-position locking handle incorporated and molded into it. The cup handle is attached to the sidewall recess by attachment members which deform in a 'twisting' or torque-like fashion when the cup handle is urged upwardly. US 2005/011854 discloses a fitment for a foodstuff container having a mouth surrounded by a rim, the fitment including an annular portion for engaging the rim surrounding the mouth of the container so as to retain the fitment on the container and a utensil moulded integrally with the annular portion to project outwards from the annular portion, wherein the utensil is connected to the annular portion by means of a hinge so as to permit the utensil to be pivoted to lie against one side of the container, and the hinge includes a toggle mechanism such that the utensil is biased away from an over-centre position. The present invention comprises a specimen container aimed at overcoming, at least in part, the abovementioned problem while still resulting in a container that is simple and inexpensive to produce in large quantities.

References to prior art in this specification are provided for illustrative purposes only and are not to be taken as an admission that such prior art is part of the common general knowledge in Australia or elsewhere.

### Summary of the Invention

According to one aspect of the present invention there is provided a specimen container comprising: a receptacle having an opening to receive a sample; a lid to engage with the receptacle and seal across the opening; an elongate handle extending from, and pivotally connected at a first end thereof to, an outer surface of the receptacle, the handle is pivotable relative to the receptacle between a first position, in which the handle extends generally downwardly from the first end parallel to the outer surface, and a second extended position; the handle includes a retaining means to retain the handle in the first position characterised in that one or more resilient members are provided to bias the handle to move towards the second position when released from the retaining means, wherein the one or more resilient members comprise resilient plastic material flexed into a curved condition when the handle is in the first position thereof such that the resilient members will tend to straighten when released and move the handle towards the second position.

Preferably, in the second position a second outer end of the handle extends upwardly of the first end.

In a preferred embodiment, the handle comprises a flat elongate member having a curvature in transverse cross section being the same as that of the outer surface of the receptacle. Further, the thickness of the handle is configured such that the handle does not protrude beyond the extents of the lid when in the first position.

The handle is preferably connected to the outer surface by a hinge means including a flexible web of material joining the handle to the receptacle.

A pair of resilient members may be provided, one on each side of the hinge means, the resilient members each comprise a portion of resilient material extending parallel to the outer surface when the handle is in the first position thereof. The retaining means may comprise a pair of lugs located between the hinge means and the resilient members when the handle is in the first position such that edges of the first end of the handle engage with recesses in the lugs to retain the handle.

Throughout the specification, unless the context requires otherwise, the word "comprise" or variations such as "comprises" or "comprising", will be understood to imply the inclusion of a stated integer or group of integers but not the exclusion of any other integer or group of integers. Likewise the word "preferably" or variations such as "preferred", will be understood to imply that a stated integer or group of integers is desirable but not essential to the working of the invention.

### Brief Description of the Drawings

The nature of the invention will be better understood from the following detailed description of several specific embodiments of the specimen container, given by way of example only, with reference to the accompanying drawings, in which:
Figure 1 is a side view of a specimen container in accordance with an embodiment of the present invention with the handle in a first position;
Figure 2 is a side view of the specimen container of Figure 1 with the handle in a second extended position;
Figure 3 is an upper perspective view of the specimen container of Figure 1 with the handle in the first position and the lid removed;
Figure 4 is an upper perspective view of the specimen container of Figure 1 with the handle in the second extended position and the lid removed;
Figure 5 is a lower perspective view of the specimen container of Figure 1 with the handle in the second extended position and the lid removed;
Figure 6 is a side cross sectional view of the specimen container of Figure 1 with the handle in the first position and the lid removed;
Figure 7 is a side cross sectional view of the specimen container of Figure 1 with the handle in the second extended position and the lid removed;
Figure 8 is a view of two of the specimen containers of Figure 1 stacked on top of each other with the handles in the first position; and
Figure 9 is a perspective view of a specimen container according to a further preferred embodiment of the present invention, with the specimen container being shown in a first condition (this embodiment does not fall within the scope of the present invention);
Figure 10 is a perspective view of the specimen container shown in Figure 9, with the specimen container being shown in a second condition;
Figure 11 is a perspective view of the specimen container shown in Figure 9, with the specimen container being shown in a third condition;
Figures 12a and 12b comprise a perspective view and a side view respectively illustrating the operation of the specimen container shown in Figure 9;
Figure 13 is a further perspective view of the specimen container shown in Figure 9; and

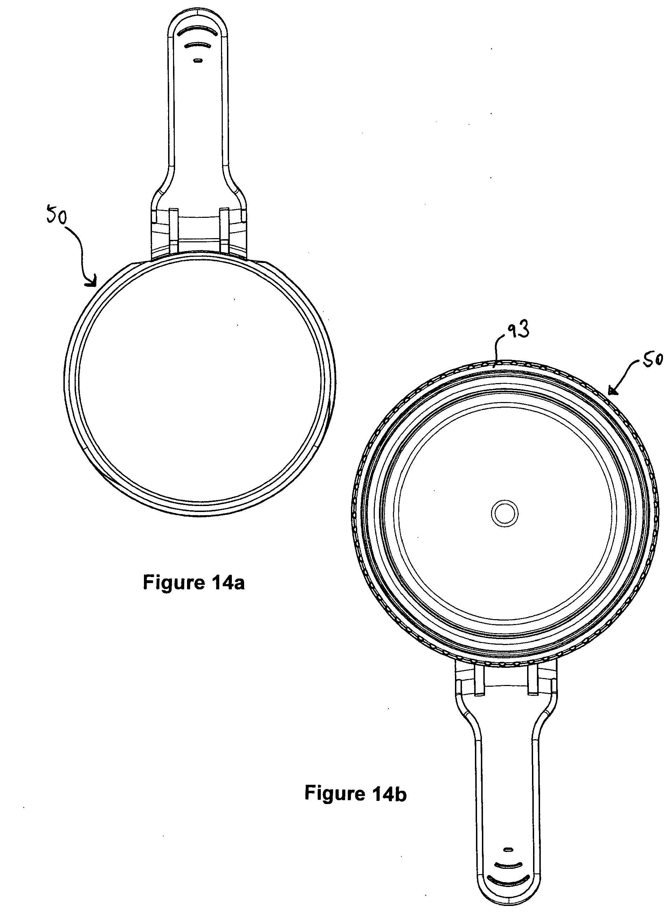

Figure 14a is a top plan view of the container of Figure 9 with the handle extended and without a lid, and Figure 14b is a top plan view of the container of Figure 9 with the handle extended and with the lid in place.

### Detailed Description of Preferred Embodiments

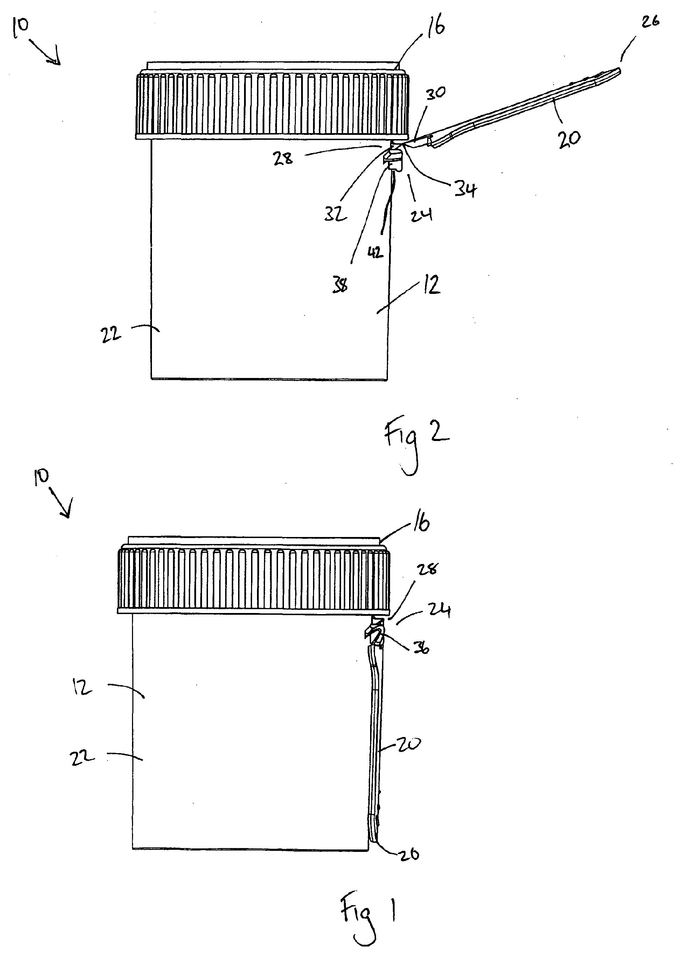

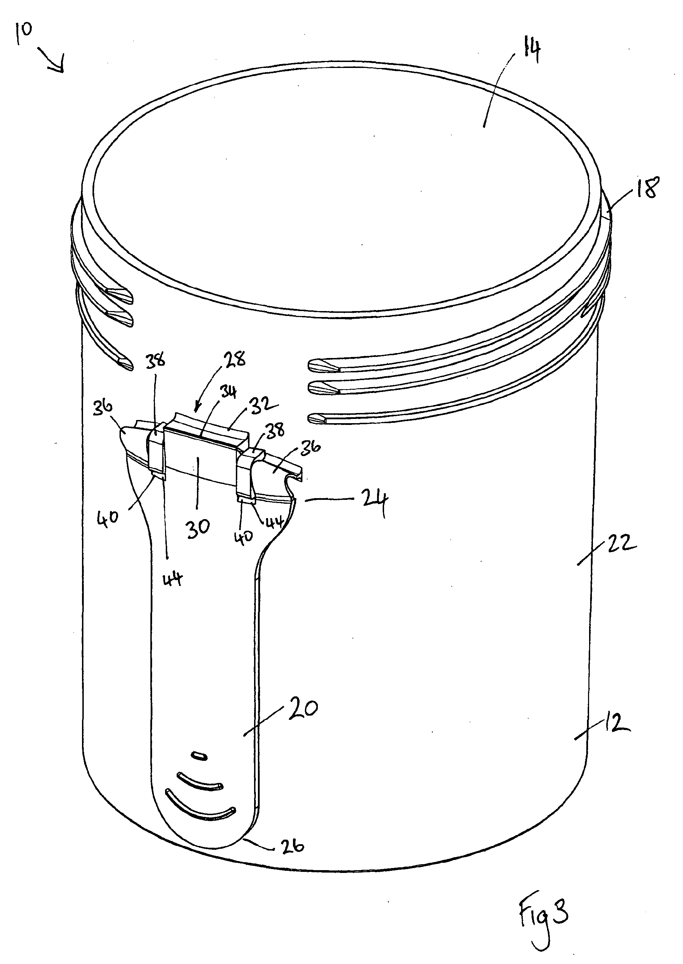

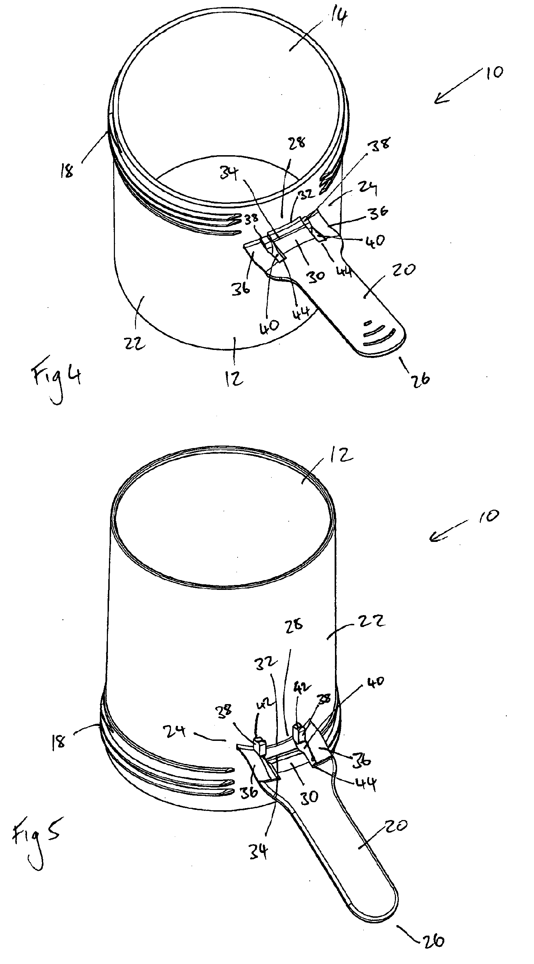

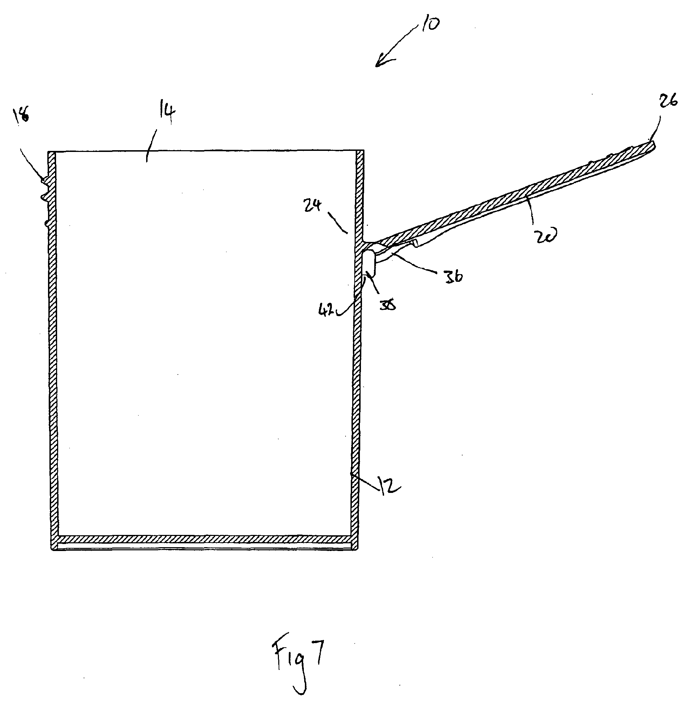

A preferred embodiment of specimen container 10 in accordance with the invention, as illustrated in Figures 1 to 8, comprises a receptacle 12 having an opening 14 for receiving a sample. The specimen container 10 also includes a lid 16 to engage with the opening 14 and seal the opening 14.

The receptacle 12 in the embodiment shown comprises a standard cylindrical container of a type known for use as a specimen container having an outer thread 18 around the opening 14. The outer thread 18 engages with an inner thread on the lid 16. The specimen container 10 is expected to be constructed from a suitable plastic material.

The specimen container 10 of the present invention includes also a handle 20 secured to an outer surface 22 of the cylindrical receptacle 12. The handle 20 is secured at a first end 24 thereof to the outer surface 22 of the receptacle 12 such that the handle 20 is pivotable relative to the receptacle 12.

The handle 20 comprises a relatively flat elongate member which is pivotable about the first end 24 thereof. The handle 20 is pivotable upwardly from a first position (as shown in Figure 1) to a second extended position (as shown in Figure 2). In the first position, the handle 20 extends generally downwardly from the first end 24, parallel to the outer surface 22. In pivoting to the second extended position, the handle 20 preferably moves through over ninety degrees such that a second outer end 26 of the handle extends slightly upwardly of the first end 24. The angle of the handle in the second extended position assists in allowing for the convenient collection of a specimen sample by a patient and also provides good support for the added weight of the specimen container 10 when it is filled with fluid.

The handle 20 also is preferably slightly arcuate in transverse cross section, with the curvature being the same as the outer surface 22 of the receptacle 12. In the first position, the handle 20 therefore rests flush against the outer surface 22. Preferably, the thickness of the handle 20 is such that it does not protrude beyond the outer extents of the lid 16 in the first position.

The pivotable connection of the first end 24 of the handle 20 with the outer surface 22 of the receptacle 12 is via a hinge means 28. The hinge means comprises a central portion 30 of the first end 24 of the handle 20 connected to a protrusion 32 on the outer surface 22 by a web 34 of the material from which the container 10 is constructed. The web 34 is sufficiently thin to be flexible such that the handle 20 can pivot about the protrusion 32.

The handle 20 also includes a retaining means to hold the handle 20 in the first position thereof and at least one resilient member 36 to bias the handle 20 to move towards the second position thereof when released from the retaining means. In the embodiment shown, there are provided two resilient members 36 provided at the first end 24 of the handle 20, one on either side of the hinge 28. Each of the resilient members 36 comprise a portion of material connected from the first end 24 of the handle 20 to the outer surface 22 of the receptacle 12. The resilient members 36 comprise resilient plastic material flexed into a curved condition when the handle 20 is in the first position thereof such that the resilient members 36 will tend to straighten when released and move the handle 20 towards the second position.

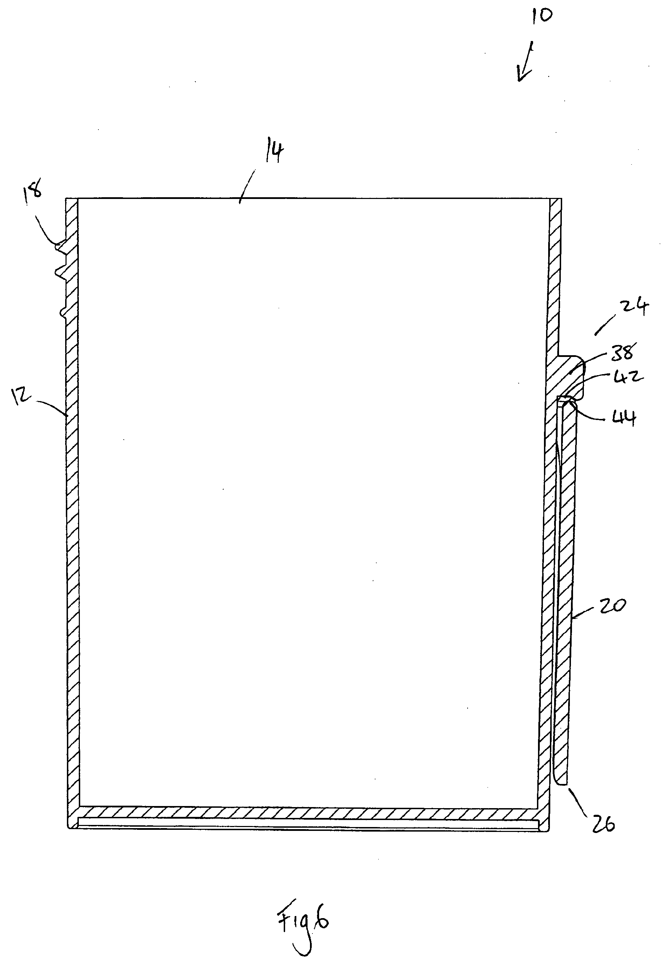

The retaining means comprises a pair of lugs 38 on the outer surface 22 of the receptacle 12. Each of the lugs 38 is received in a gap 40 between the hinge 28 and one of the resilient members 36 when the handle 20 moves to the first position. Each of the lugs 38 includes a recess 42 on a lower side thereof. The recesses 42 are positioned such that inner edges 44 of the gaps 40 engage in the recesses 42 (as can be seen in Figure 6) when the handle 20 is in the first position.

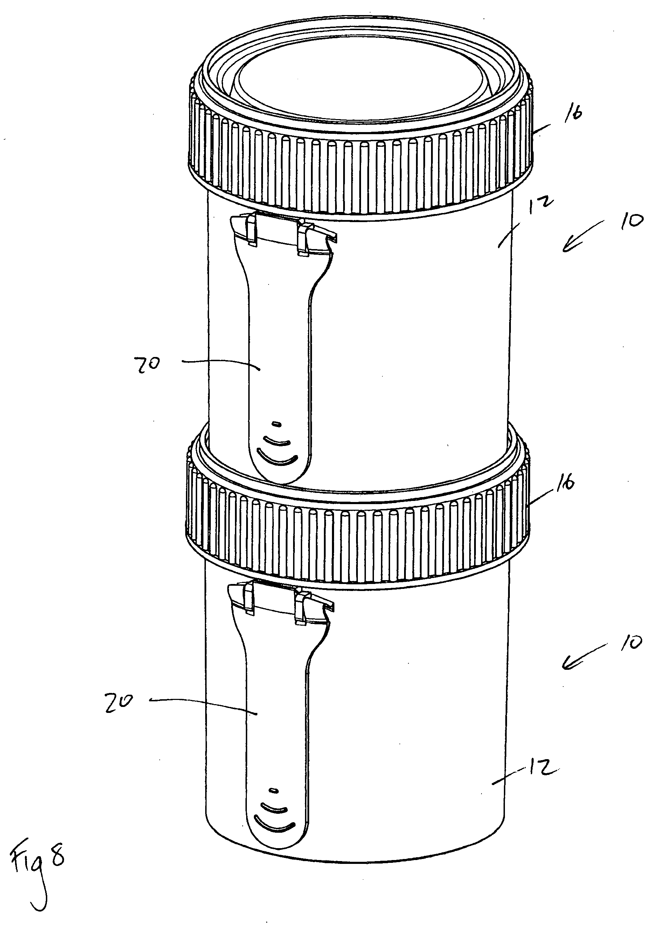

It is expected that the receptacle 12 and handle 20 of the specimen container 10 will be moulded as a single plastic unit resulting with the handle 20 in an extended position. The handle 20 will then be flexed about its pivotal connection and moved to the first position in which the lugs 38 engage with the handle 20 and retain the handle 20 in place. The specimen container is then supplied to the end user in this configuration. The lid 16 of the container 10 is preferably shaped to receive the lower end of the receptacle 12 so that the containers 10 can be stacked for transport, as shown in Figure 8.

When required for use, the outer end 26 of the handle 20 is pulled outwards to disengage from the lugs 38. The resilient members 36 then cause the handle 20 to pivot outwardly and remain held in the second extended position. The user can then hold the specimen container 10 by the second end 26 of the handle 20 when providing a sample to ensure their hands are well clear of the receptacle.

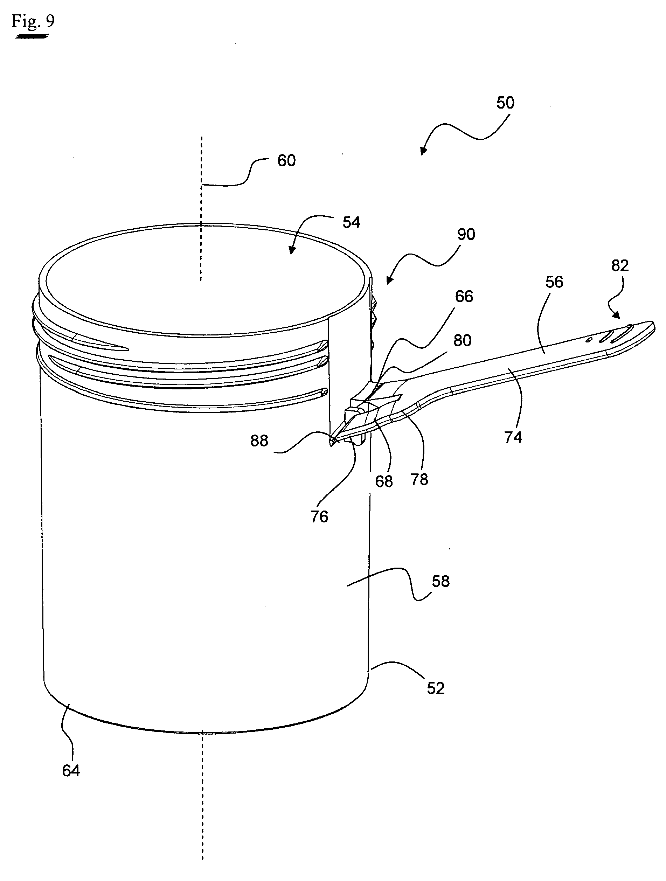

Referring now to Figure 9 there is shown a specimen container 50 according to a further preferred embodiment of the present invention. The specimen container 50 is considered to be advantageous as it is relatively readily moulded, manufactured, packaged and employed.

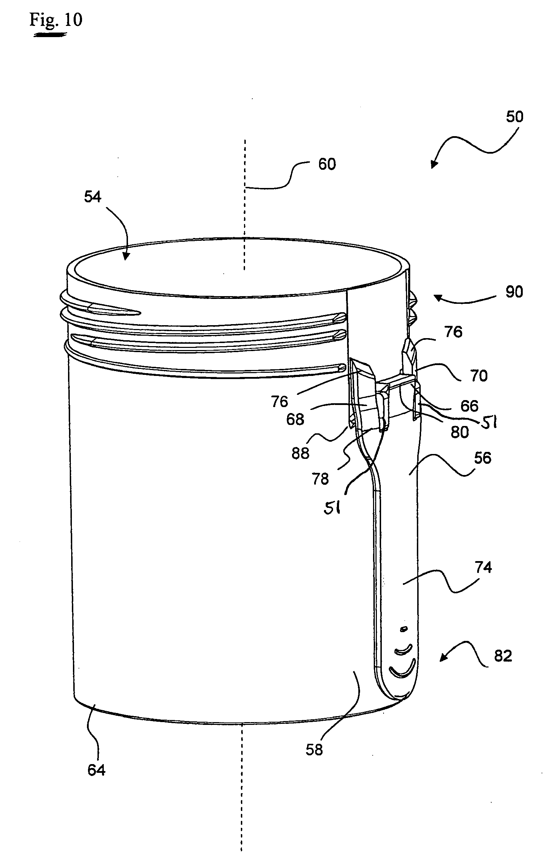

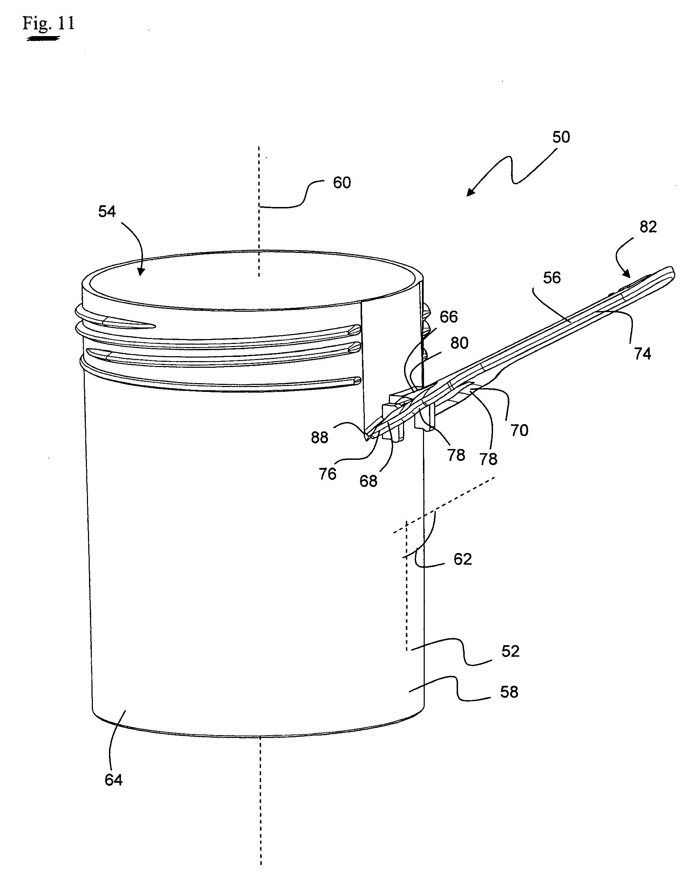

As shown in Figure 9 the specimen container 50 includes a body 52 having an opening 54 for receiving a liquid. A handle 56 is connected to the body 52, and a pair of lugs 51 are provided on the outer surface 58 of the body 52. The handle 56 is moveable between a retracted position shown in Figure 10 and an extended position shown in Figure 11. In the retracted position, shown in Figure 10, the handle 54 extends along the surface 58 of the body 52 substantially in line with a longitudinal axis 60 thereof. In the extended position, shown in Figure 11, the handle 56 is inclined at an angle 62, relative to the body 52, so as to extend upwardly and outwardly away from a base 64 of the body 52.

In the extended position the handle 56 is adapted to engage the body 52 so as to bear against the surface 58. This allows a person to hold the handle 56 in preparation for liquid to be received through the opening 54 of the body 52.

As shown in Figure 11 a pivotal connection 66 is provided between the body 52 and the handle 56. A first arm 68 and a second arm 70 of the handle 56 are spaced either side of the pivotal connection 66. The handle 56 has a body 74.

The first arm 68 and the second arm 70 each have a free end 76 and are connected to the remainder of the handle 56 at connecting regions 78. The connecting regions 78 are of a similar thickness to the body 74 of the handle 56. This is considered to be advantageous for reasons relating to manufacture.

With the arrangement, the pivotal connection 66 is provided as a hinge 80. In the extended position shown in Figure 11, the first arm 68 and the second arm 70 are disposed to present the free ends 76 to bear against the surface 58 of the body 52. As would be apparent, holding the handle 56 with a thumb and an index finger at position 82, and pouring fluid through the opening 54 will operate to urge the body 52 to rotate about the hinge line of the hinge 80. This causes free ends 76 of the first arm 68 and the second arm 70 to bear against the surface 58 to keep the body 52 upright, and resist movement of the handle 56 away from the extended position shown in Figure 11. The free ends 76 bear advantageously against raised portions 88 as is described in more detail below.

As shown in Figure 10, the free ends 76 of the first arm 68 and the second arm 70 extend above the hinge 80 when in the retracted position. In order to accommodate movement of the free ends 76 from below the hinge 80 to above the hinge 80 (and vice versa), the arms 68, 70 are flexibly connected to the body 74 of the handle 56. In this arrangement, the arms 68, 70 are relatively rigid and function as cantilevers flexing primarily at the connecting regions 78. In other arrangements the arms 68, 70 flex appreciably along a much greater portion of their lengths.

In order to provide a snapping action when moving the handle 56 from the extended position to the retracted position, the two arms 68, 70 are slightly longer in length than the central portion 83 of the handle which is joined to the hinge 80, providing a snapping action when moving the free ends 76 from below the hinge 80 to above the hinge 80.

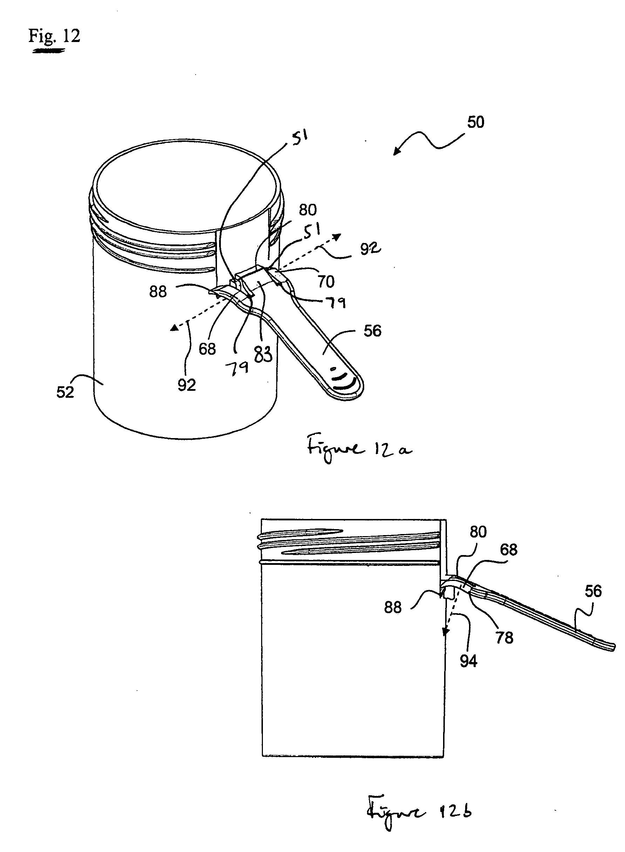

As shown in Figure 12 the raised portions 88 are of a similar width to each of the arms 68, 70. Figure 12 illustrates that a person moving the handle 56 from the extended position to the retracted position will cause downwards and sidewards flexing of the arms 68, 70 at the connecting regions 78. Flexing in the sidewards direction is illustrated by arrows 92 and flexing in the downwards direction is illustrated by arrows 94. The arms 68, 70 flex primarily at their connecting regions 78 until they have moved substantially away from the raised portions 88 and then snap upwardly. A pair of slots 79 separates the arms 68, 70 from the hinge 80.

During the snapping action the arms 68, 70 splay outwards and then move above the hinge 80. This is assisted by the free ends 76 having outwardly inclined surfaces that point upwardly when in the retracted position. The free ends 76 are chamfered to engage with the projections 88 as shown in Figure 11 and then snap upwardly through the position shown in Figure 12.

In the arrangement, the specimen container 50 is manufactured in a moulding process to form a unitary piece of plastics material as illustrated in Figure 9. The first arm 68 and the second arm 70 are moulded so as to be joined to the raised portions 88. Following this, a cutting action is performed to separate the first arm 68 and the second arm 70 from the raised portions 88.

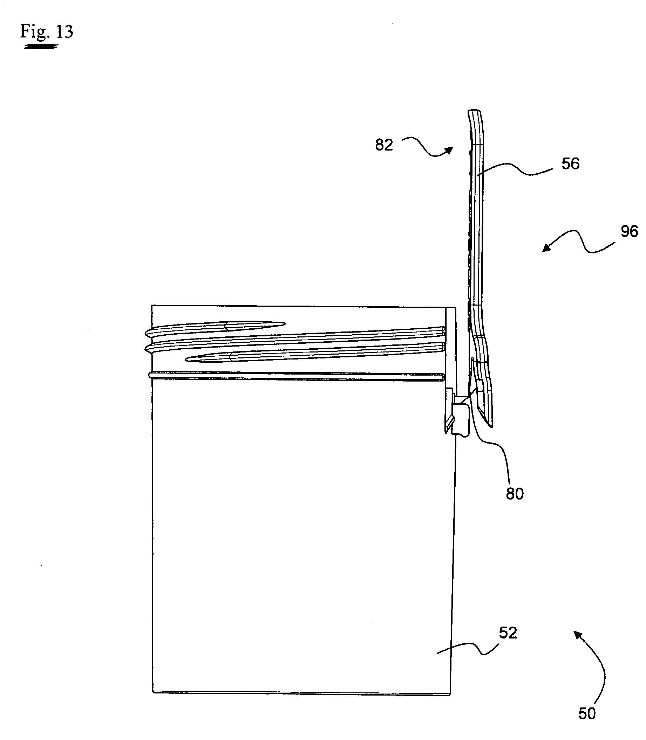

As shown in Figure 13, the handle 56 is moveable to an overextended position 96 lying beyond the extended position. The hinge 80 operates between the overextended position 96 and the extended position to bias the handle 56 towards the extended position shown in Figure 11.

It is to be appreciated that when in the retracted position, moving the handle 56 towards the extended position will cause the first and second arms 68, 70 to bear against an upper portion 90 of the body 52 shown in Figure 10. Before the angle between the body 52 and the handle 56 reaches about 90 degrees, the arms 68, 70 bear against the upper portion 90 to bias the handle 56 towards the retracted position.

The arms 68, 70 are configured for being held underneath a cap 93 when the cap is screwed onto the upper portion 90. This assists with keeping the handle 56 in the retracted position during transportation. Advantageously the handle 56 lies within the margins of the rim of the cap when in the retracted position.

By way of overview, the method of manufacture of the container 50 comprises moulding the container 50 in a position in which the handle 56 extends from the body 52 and is joined to the body 52 by an upper join at the hinge 80 and two lower joins at the arms 68, 70. Following this the two lower joins are cut to provide the handle 56 with abutment portions at the end of the arms 68, 70. The handle 56 is adapted to pivot about the hinge 80 with the arms 68, 70 being adapted to flex as the handle 56 is rotated to push the end of the abutment portions from below the pivot to above the pivot, and vice versa.

Although not shown the handle 56 includes a slight ridge along its length to strengthen the rigidity of the handle. As described, the specimen container 50 is considered to be advantageous for the reasons discussed above. These reasons include those relating to manufacture, packaging and use.

It will be readily apparent to persons skilled in the relevant arts that various modifications and improvements may be made to the foregoing embodiments, in addition to those already described, without departing from the basic inventive concepts of the present invention. Therefore, it will be appreciated that the scope of the invention is not limited to the specific embodiments described.

## Claims

1. A specimen container (10) comprising: a receptacle (12) having an opening (14) to receive a sample; a lid (16) to engage with the receptacle (12) and seal across the opening (14); an elongate handle (20) extending from, and pivotally connected at a first end (24) thereof to, an outer surface of the receptacle (12), the handle (20) is pivotable relative to the receptacle (12) between a first position, in which the handle (20) extends generally downwardly from the first end (24) parallel to the outer surface (22), and a second extended position; the handle (20) includes a retaining means (38) to retain the handle (20) in the first position **characterised in that** one or more resilient members (36) are provided to bias the handle (20) to move towards the second position when released from the retaining means (38), wherein the one or more resilient members (36) comprise resilient plastic material flexed into a curved condition when the handle (20) is in the first position thereof such that the resilient members (36) will tend to straighten when released and move the handle (20) towards the second position.

2. A specimen container (10) as defined in claim 1, wherein in the second position a second outer end (26) of the handle (20) extends upwardly of the first end (24).

3. A specimen container (10) as defined in claim 1 or claim 2, wherein the handle (20) comprises a flat elongate member having a curvature in transverse cross section being the same as that of the outer surface (22) of the receptacle (12).

4. A specimen container (10) as defined in any one of claims 1 to 3, wherein the thickness of the handle (20) is configured such that the handle (20) does not protrude beyond the extents of the lid (16) when in the first position.

5. A specimen container (10) as defined in any one of the preceding claims, wherein the handle (20) is connected to the outer surface (22) by a hinge means (28) including a flexible web (34) of material joining the handle (20) to the receptacle (12).

6. A specimen container (10) as defined in claim 5, wherein a pair of resilient members (36) is provided, one on each side of the hinge means (28), the resilient members (36) each comprise a portion of resilient material extending parallel to the outer surface (22) when the handle (20) is in the first position thereof.

7. A specimen container (10) as defined in claim 6, wherein the retaining means comprises a pair of lugs (38) located between the hinge means (28) and the resilient members (36) when the handle (20) is in the first position such that edges of the first end of the handle (20) engage with recesses (42) in the lugs (38) to retain the handle (20).

## Patentansprüche

1. Probenbehälter (10), umfassend: ein Behältnis (12) mit einer Öffnung (14) zur Aufnahme einer Probe; einen Deckel (16) für einen Eingriff mit dem Behältnis (12) und mit einem Verschluss über der Öffnung (14); einem länglichen Handstück (20), das sich von einem ersten Ende (24) des Behältnisses (12) zu einer äußeren Oberfläche des Behältnisses erstreckt und an dem ersten Ende drehbar verbunden ist, wobei das Handstück (20) im Verhältnis zu dem Behältnis (12) drehbar ist zwischen einer ersten Position, an der sich das Handstück (20) allgemein von dem ersten Ende (24) parallel zu der äußeren Oberfläche (22) nach unten erstreckt, und einer zweiten, erweiterten Position; wobei das Handstück (20) eine Halteeinrichtung (38) aufweist, um das Handstück (20) an der ersten Position zu halten, **dadurch gekennzeichnet, dass** ein oder mehrere elastische Elemente (36) vorgesehen sind, um das Handstück (20) so vorzubelasten, dass es sich an die zweite Position bewegt, wenn es von der Halteeinrichtung (38) freigegeben wird, wobei das eine oder die mehreren elastischen Elemente (36) einen elastischen Kunststoff umfassen, der in einen gekrümmten Zustand gebogen wird, wenn sich das Handstück (20) an seiner ersten Position befindet, so dass die elastischen Elemente (36) dazu neigen, sich gerade zu richten, wenn sie freigegeben werden und das Handstück (20) in Richtung der zweiten Position zu bewegen.

2. Probenbehälter (10) nach Anspruch 1, wobei sich an der zweiten Position ein zweites äußeres Ende (26) des Handstücks (20) von dem ersten Ende (24) nach oben erstreckt.

3. Probenbehälter (10) nach Anspruch 1 oder 2, wobei das Handstück (20) ein flaches, längliches Element mit einer Krümmung umfasst, die im Querschnitt der Krümmung der äußeren Oberfläche (22) des Behältnisses (12) entspricht.

4. Probenbehälter (10) nach einem der Ansprüche 1 bis 3, wobei die Dicke des Handstücks (20) so konfiguriert ist, dass das Handstück (20) an der ersten Position nicht über die Abmessungen des Deckels (16) vorsteht.

5. Probenbehälter (10) nach einem der vorstehenden Ansprüche, wobei das Handstück (20) mit der äußeren Oberfläche (22) verbunden ist durch eine Gelenkeinrichtung (28) mit einer flexiblen Materialbahn (34), welche das Handstück (20) mit dem Behältnis (12) verbindet.

6. Probenbehälter (10) nach Anspruch 5, wobei ein Paar elastischer Elemente (36) vorgesehen ist, eines auf jeder Seite der Gelenkeinrichtung (28), wobei die elastischen Elemente (36) jeweils einen Teil des elastischen Materials umfassen, das sich parallel zu der äußeren Oberfläche (22) erstreckt, wenn sich das Handstück (20) an seiner ersten Position befindet.

7. Probenbehälter (10) nach Anspruch 6, wobei die Halteeinrichtung ein Paar von Ansätzen (38) umfasst, die zwischen der Gelenkeinrichtung (28) und den elastischen Elementen (36) angeordnet sind, wenn sich das Handstück (20) an der ersten Position befindet, so dass Kanten des ersten Endes des Handstücks (20) mit Vertiefungen (42) in den Ansätzen (38) eingreifen, um das Handstück (20) zu halten.

## Revendications

1. Contenant à échantillon (10) comprenant : un réceptacle (12) ayant une ouverture (14) pour recevoir un échantillon ; un couvercle (16) pour venir en prise avec le réceptacle (12) et sceller l'ouverture (14) ; une poignée allongée (20) s'étendant de, et reliée de façon pivotante au niveau d'une première extrémité (24) de celle-ci à une surface externe du réceptacle (12), la poignée (20) pouvant pivoter par rapport au réceptacle (12) entre une première position, dans laquelle la poignée (20) s'étend généralement vers le bas à partir de la première extrémité (24) parallèlement à la surface externe (22), et une seconde position déployée ; la poignée (20) comprenant un moyen de retenue (38) pour retenir la poignée (20) dans la première position, **caractérisé en ce qu'**au moins un élément élastique (36) est fourni pour rappeler la poignée (20) afin qu'elle avance vers la seconde position lorsqu'elle est relâchée par le moyen de retenue (38), l'au moins un élément élastique (36) comprenant une matière plastique élastique fléchie dans un état courbé lorsque la poignée (20) est dans sa première position, de sorte que les éléments élastiques (36) tendent à se redresser en cas de libération et à déplacer la poignée (20) vers la seconde position.

2. Contenant à échantillon (10) selon la revendication 1, à la seconde position, une seconde extrémité externe (26) de la poignée (20) s'étendant vers le haut de la première extrémité (24).

3. Contenant à échantillon (10) selon la revendication 1 ou 2, la poignée (20) comprenant un élément allongé plat ayant une courbure dans sa section transversale identique à celle de la surface externe (22) du réceptacle (12).

4. Contenant à échantillon (10), selon l'une quelconque des revendications 1 à 3, l'épaisseur de la poignée (20) étant conçue de sorte que la poignée (20) ne fasse pas saillie au-delà de l'étendue du couvercle (16) dans sa première position.

5. Contenant à échantillon (10) selon l'une quelconque des revendications précédentes, la poignée (20) étant reliée à la surface externe (22) par un moyen charnière (28) comprenant une âme souple (34) de matériau reliant la poignée (20) au réceptacle (12).

6. Contenant à échantillon (10) selon la revendication 5, comprenant une paire d'éléments élastiques (36), un de chaque côté du moyen charnière (28), les éléments élastiques (36) comprenant chacun une partie de matériau élastique s'étendant parallèlement à la surface externe (22) lorsque la poignée (20) est dans sa première position.

7. Contenant à échantillon (10) selon la revendication 6, le moyen de retenue comprenant une paire d'ergots (38) située entre le moyen charnière (28) et les éléments élastiques (36) lorsque la poignée (20) est dans la première position de sorte que les bords de la première extrémité de la poignée (20) viennent en prise avec des évidements (42) dans les ergots (38) pour retenir la poignée (20).
